**Europäisches Patentamt**

**European Patent Office**

**Office européen des brevets**

(19)

(11) Veröffentlichungsnummer: **0 449 784 A1**

(12) # EUROPÄISCHE PATENTANMELDUNG

(21) Anmeldenummer: **91810234.4**

(22) Anmeldetag: **28.03.91**

(51) Int. Cl.$^5$: **A61B 1/26, A61B 17/24**

(30) Priorität: **28.03.90 CH 1025/90**

(43) Veröffentlichungstag der Anmeldung:
**02.10.91 Patentblatt 91/40**

(84) Benannte Vertragsstaaten:
**AT DE DK FR GB IT SE**

(71) Anmelder: **Baumann, Peter**
**Scherzligweg 18**
**CH-3600 Thun (CH)**

(72) Erfinder: **Baumann, Peter**
**Scherzligweg 18**
**CH-3600 Thun (CH)**

(74) Vertreter: **AMMANN PATENTANWAELTE AG**
**BERN**
**Schwarztorstrasse 31**
**CH-3001 Bern (CH)**

(54) **Intubationsspatel.**

(57)  Das Spatelprofil (2) des Intubationsspatels weist in einem mittleren Bereich einen nach innen geneigten Flansch (4) auf, welcher längs einer Kante (6) an einen Steg (5) anschliesst. Das schnabelförmige Spatelprofil ist vom Griff aus über etwa halbe Länge in einem Sinne, anschliessend über etwa einen Viertel der Länge entgegengesetzt und etwa im äussersten Viertel der Länge wieder in einem Sinne gekrümmt. Diese besondere Formgebung des Spatelprofils gestattet bei der Intubierung eine besonders wirksame Verdrängung der Zunge und eine gute Sicht in den Kehlkopfbereich, was ein rasches, sicheres Arbeiten begünstigt.

EP 0 449 784 A1

FIG.1

Die vorliegende Erfindung betrifft einen Intubationsspatel (Laryngoskop) dessen gekrümmtes Spatelprofil einen äusseren und einen inneren Flansch und einen die Flansche verbindenden Steg aufweist, wobei der innere Flansch zum Niederhalten der Zunge bestimmt ist. Intubationsspatel dieser Art sind bekannt, und es ist ebenfalls bekannt, dass in vielen Fällen, insbesondere bei gewissen Abnormitäten im Körperbau des Patienten, Schwierigkeiten entstehen, weil das Spatelprofil selbst die Sicht auf den Kehlkopf zu stark behindert.

Aus der DE-A-31 43 619 ist es bekannt, zur Milderung dieser Schwierigkeiten dem Spatelprofil eine flache V-Form zu erteilen und auf dem äusseren, geraden Teil desselben ein Prisma anzubringen, welches die Beobachtungsmöglichkeiten verbessern soll. Diese Ausführung ist jedoch aufwendig und gewährleistet auch keine optimale Beobachtung.

Ziel vorliegender Erfindung ist es, durch einfache, neuartige Formgebung des Spatelprofils die erwähnten Schwierigkeiten zu beheben. Eine erste Massnahme besteht darin, den inneren Flansch des Spatelprofils in einem Mittelteil einwärts zu neigen. Dadurch kann bei eingeführtem Spatel durch Zug an dessen Griff die Zunge des Patienten besonders wirksam nach unten und zur Seite verdrängt werden, so dass schliesslich das Spatelprofil im Mund tiefer liegt und in allen Fällen eine gute Beobachtung des Kehlkopfes gewährleistet ist. Nicht nur diese gute Sicht, sondern auch die tiefe Lage des Spatelprofils im Mund des Patienten erleichtern das Intubieren.

Eine zweite, vorzugsweise in Kombination mit der oben erwähnten, erfindungsgemäss getroffenen Massnahme besteht darin, dass die Krümmung des Spatelprofils im Mittelteil desselben geringer bis entgegengesetzt zur Krümmung in den Endteilen des Spatelprofils ist. Diese Formgebung, insbesondere in Kombination mit der erwähnten Neigung des inneren Flansches, resultiert in einem optimalen Verdrängen bzw. Niederhalten der Zunge im kritischen Bereich.

Die Erfindung wird nun anhand eines in der Zeichnung dargestellten Ausführungsbeispiels näher erläutert.

Fig. 1 ist eine Seitenansicht des Intubationsspatels,

die Fig. 2 bis 8 zeigen Querschnitte des Spatelprofils in den Schnittebenen II bis VIII und

Fig. 9 zeigt die Anwendung des Intubationsspatels.

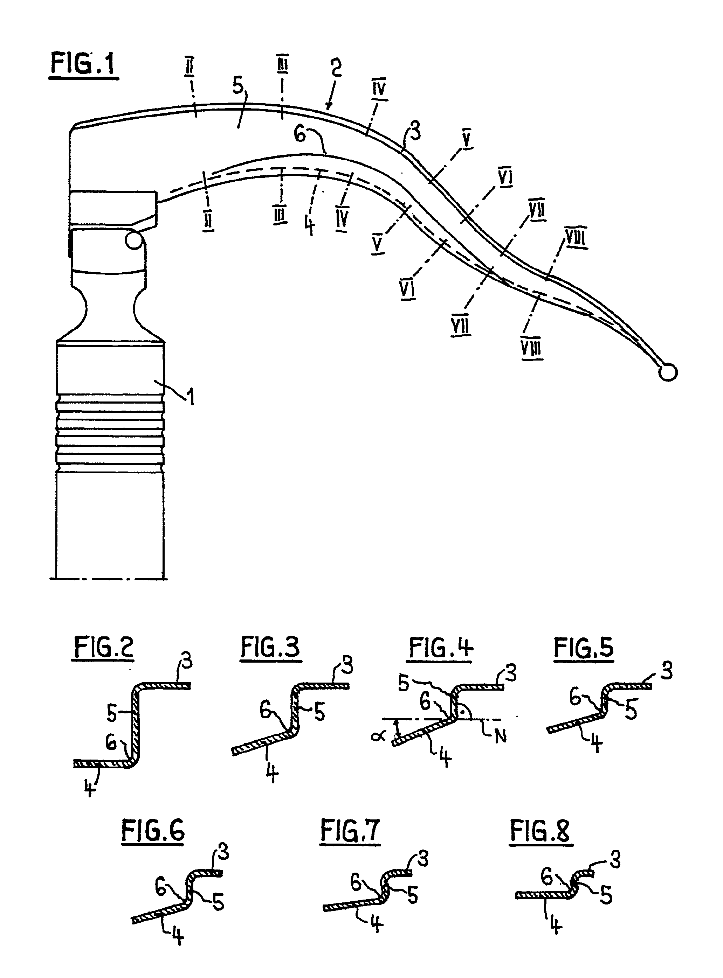

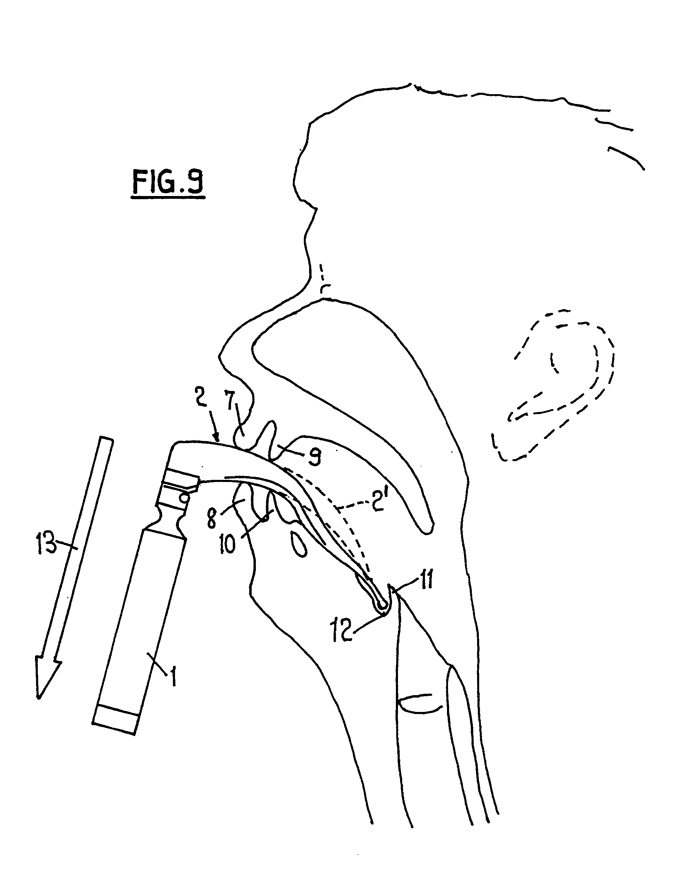

Der in Fig. 1 dargestellte Intubationsspatel weist einen zylindrischen Griff 1 auf, mit welchem ein schnabelförmiges Spatelprofil 2 gelenkig verbunden ist. Bei der dargestellten Betriebslage steht der Griff 1 etwa senkrecht zum Verlauf des Spatelprofils an seinem mit dem Griff verbundenen Ende. Wie die Fig. 2 bis 8 zeigen, weist das Spatelprofil an seinen Enden etwa Z-Form auf, mit einem äusseren Flansch 3, einem inneren Flansch 4 und einem dieselben verbindenden, etwa rechtwinklig dazu stehenden Steg 5. Wie die Fig. 2 bis 8 ferner zeigen, bleibt die Länge des inneren Flansches 4 über die ganze Länge des Spatelprofils etwa gleich, während die Länge des Flansches 3 bzw. die Höhe des Stegs 5 bis zum freien Ende des Spatelprofils allmählich abnehmen. In einem Bereiche, der sich über etwa zwei Drittel der Länge des Spatelprofils erstreckt, ist der innere Flansch 4 nach innen geneigt und zwar von der Stelle des Schnittes II bis etwa zur Stelle des Schnittes VII. Dieser Bereich ist in Fig. 1 durch den Verlauf der Kante 6 angedeutet.

In diesem Bereich nimmt die Neigung des inneren Flansches 4 von Null bis zu einem Maximum stetig zu und dann wieder ab, wobei dieses Maximum etwa in der Mitte des Bereiches zwischen den Schnitten IV und V liegt. Der maximale Neigungswinkel $\alpha$ des unteren Flansches 4 gegenüber einer Normalen N zur Ebene des Steges 5 beträgt beispielsweise etwa 30°. Man kann auch annehmen, dass diese maximale Neigung etwa bei halber Länge des Spatelprofiles liegt.

Eine weitere Besonderheit des Spatelprofils liegt im speziellen Krümmungsverlauf desselben. Das Profil ist vom Griff aus über etwa halbe Länge in einem Sinne, anschliessend über etwa einen Viertel der Länge entgegengesetzt und etwa im äussersten Viertel der Länge wieder in einem Sinne gekrümmt. Es ergibt sich damit eine Einbuchtung in der äusseren Hälfte gegenüber bekannten, über die ganze Länge im gleichen Sinne gekrümmten Spatelprofilen. In Fig. 9 ist in gestrichelten Linien die Form eines üblichen Spatelprofils 2' zum Vergleich mit dem Spatelprofil nach dem Ausführungsbeispiel angedeutet.

Zur Intubation wird das Spatelprofil gemäss Fig. 9 eingeführt, in welcher die Lippen 7, 8, die Zähne 9, 10, der Kehlkopfdeckel 11 und die Furche 12 angedeutet sind. Nachdem das Spatelprofil in dieser Weise eingeführt ist, wird am Griff 1 nach abwärts gezogen, um den Mund zu öffnen und die Intubation vorzunehmen. Dabei wird die Zunge dank der Neigung des inneren Flansches 4 wirksam nach unten und zur Seite gedrängt, so dass das Spatelprofil verhältnismässig tief in der Mundhöhle liegt, und dank der besonderen Krümmung bzw. Formgebung des Spatelprofils ist die Sicht in den Kehlkopfbereich besonders gut, was ein rasches und sicheres Arbeiten erlaubt.

## Patentansprüche

1. Intubationsspatel dessen gekrümmtes Spatelprofil (2) einen äusseren (3) und einen inneren (4) Flansch und einen die Flansche verbindenden Steg (5) aufweist, wobei der innere Flansch (4) zum Niederhalten der Zunge bestimmt ist, dadurch gekennzeichnet, dass in einem Mittelteil (6) des Spatelprofils der innere Flansch (4) einwärts geneigt ist.

2.  Spatel nach Anspruch 1, dadurch gekennzeichnet, dass die Neigung des inneren Flansches (4) je von den Enden des Mittelteils (6) bis zu einem Maximum (α) zunimmt.

3.  Spatel nach Anspruch 2, dadurch gekennzeichnet, dass die maximale Neigung (α) etwa 30° bezüglich einer Normalen (N) zur Stegebene (5) beträgt.

4.  Spatel, insbesondere nach einem der Ansprüche 1 bis 3, dadurch gekennzeichnet, dass die Krümmung des Spatelprofils im Mittelteil (6) geringer bis entgegengesetzt zur Krümmung in den Endteilen des Spatelprofils ist.

5.  Spatel nach Anspruch 4 mit einem Griff (1) am einen Ende des Spatelprofils ( 2 ), dadurch gekennzeichnet, dass das Spatelprofil vom Griff aus über etwa halbe Länge in einem Sinne, anschliessend über etwa einen Viertel der Länge entgegengesetzt, und etwa im äussersten Viertel der Länge wieder in einem Sinne gekrümmt ist.

6.  Spatel nach Anspruch 5, dadurch gekennzeichnet, dass der innere Flansch (4) über etwa zwei Drittel der Länge des Spatelprofils (2) geneigt ist, wobei die maximale Neigung (α) etwa bei halber Länge des Spatelprofils liegt.

FIG.1

FIG.2   FIG.3   FIG.4   FIG.5

FIG.6   FIG.7   FIG.8

FIG.9

Europäisches
Patentamt

**EUROPÄISCHER RECHERCHENBERICHT**

Nummer der Anmeldung

EP 91 81 0234

| | EINSCHLÄGIGE DOKUMENTE | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | EP-A-0 125 687 (GUSTAV MÜLLER GmbH & CO. KG)<br>* Seite 7, Zeilen 1-17; Seite 13, Zeile 5 - Seite 15, Zeile 2; Abbildungen 2a,2b * <br>--- | 1 | A 61 B 1/26<br>A 61 B 17/24 |
| A | US-A-2 657 691 (N. NORDSTROM)<br>* Spalte 3, Zeilen 3-26; Ansprüche 1,3; Abbildungen 1,2,5 *<br>--- | 1,4 | |
| A,D | DE-A-3 143 619 (AVULUNGA PTY LTD)<br>* Seite 9, Zeile 25 - Seite 10, Zeile 30; Seite 12, Zeilen 21-28; Seite 15, Zeile 28 - Seite 16, Zeile 15; Abbildungen 1-7 *<br>----- | 1 | |
| | | | **RECHERCHIERTE SACHGEBIETE (Int. Cl.5)**<br><br>A 61 B<br>A 61 M |

Der vorliegende Recherchenbericht wurde für alle Patentansprüche erstellt

| Recherchenort | Abschlußdatum der Recherche | Prüfer |
|---|---|---|
| DEN HAAG | 28-06-1991 | FONTENAY P.H.E.V. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.......................................................................................................
& : Mitglied der gleichen Patentfamilie, übereinstimmendes
    Dokument

EPO FORM 1503 03.82 (P0403)